# EUROPEAN PATENT APPLICATION

(11) **EP 4 751 766 A1**
(43) Date of publication of application: **03.06.2026**
(21) Application number: 24215910.1
(22) Date of filing: 27.11.2024
(51) Int. Cl.: A61N 1/39

(54) **HANDHELD DEVICE CONVERSION INTO RESUSCITATION HUB**

(71) Applicant: Koninklijke Philips N.V., 5656 AE Eindhoven (NL)
(72) Inventor: LIU, Chenguang, 5656 AG Eindhoven (NL); JORGENSON, Dawn Blilie, 5656 AG Eindhoven (NL)
(74) Representative: Philips Intellectual Property & Standards

(57) **Abstract**

A defibrillator (200) employing a shock delivery circuit (207) and a defibrillation controller (204) for controlling an administration of a cardiopulmonary resuscitation procedure on a heart of a patient by the defibrillator (200) in accordance with a rescue protocol including an interruptible cardiopulmonary resuscitation protocol, an uninterruptible resuscitation protocol and a shock protocol. In operation, the defibrillation controller (204) controls a series of sequential executions of the interruptible cardiopulmonary resuscitation protocol and the shock protocol in response to a preceding uninterrupted defibrillating shock delivery to the heart of the patient by the shock delivery circuit (207), and controls a sequential execution of the uninterruptible cardiopulmonary resuscitation protocol and the shock protocol when at least two sequential executions of the interruptible cardiopulmonary resuscitation protocol and the shock protocol results in a maximum number of consecutive interrupted defibrillating shock deliveries to the heart of the patient by the shock delivery circuit (207).

## Description

### FIELD OF THE INVENTION

The present disclosure generally relates to a resuscitation rescue effort by a responder or responders of a patient experiencing cardiac arrest. The present disclosure particularly relates to a conversion of a handheld device (e.g., a mobile phone and a tablet) into a resuscitation hub.

### BACKGROUND OF THE INVENTION

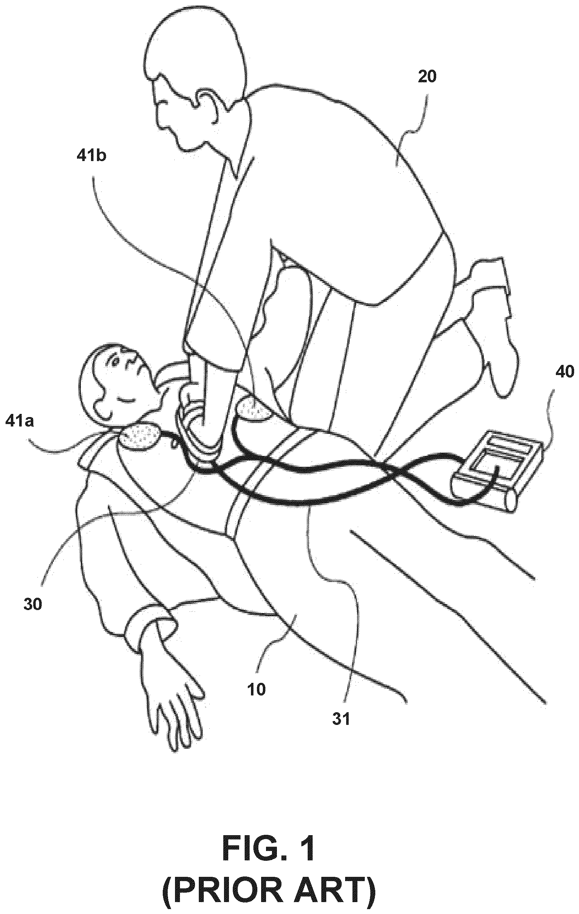

As shown in FIG. 1, a defibrillator 40 is attached to a patient 10 by electrodes 41a and 41b by a responder 20. Defibrillator 40, as known in the art of the present disclosure, can be used to deliver defibrillating shocks to patient 10 when patient 10 is suffering from cardiac arrest. More specifically, defibrillator 40 can deliver a high-voltage impulse to the heart of patient 10 in order to restore organized rhythm and contractile function for the heart of patient 10 when patient 10 is experiencing an arrhythmia (e.g., ventricular fibrillation (VF) or ventricular tachycardia (VT)) that is not accompanied by spontaneous circulation.

Still referring to FIG. 1, defibrillator 40 can be manual defibrillator, an automatic external defibrillator or a semi-automatic external defibrillator whereby electrodes 41a and 41b are applied across the chest of patient 10 by responder 20 as shown in order to acquire an electrocardiogram (ECG) signal from the patient's heart.

Defibrillator 40, when designed as a manual defibrillator, graphically display the ECG for responder 20 to analyze cardiac rhythm(s) of the ECG to determine if a defibrillation is necessary. Defibrillator 40, when designed as an external defibrillator, automatically analyzes cardiac rhythm(s) of the ECG to determine if defibrillation is necessary. If defibrillator 40 detects a VF cardiac rhythm or another shockable cardiac rhythm of the ECG and defibrillator 40 is designed as an automatic external defibrillator, then defibrillator 40 automatically delivers a defibrillating shock in an attempt to resuscitate patient 10. If defibrillator 40 detects a VF cardiac rhythm or another shockable cardiac rhythm and defibrillator 40 in the ECG and is designed as a semi-automatic external defibrillator, then defibrillator 40 signals responder 20 that a shock is advised, whereby the responder 20 can press a shock button on defibrillator 40 to deliver a defibrillating shock in an attempt to resuscitate patient 10.

As shown in FIG. 1, a CPR coaching device 30 can be coupled to defibrillator 40 by an electrical cable 31 to provide defibrillator 40 with physiological information obtained by sensors contained in the CPR coaching device 30 as known in the art of the present disclosure. Electrical cable 31 provides power for electronic components in CPR coaching device 30 and couples compression related signals to defibrillator 40 (e.g., compression depth, compression rate, chest release and recoil), whereby the physiological information indicated by the signals can be used to issue audible CPR instructions through the loudspeaker of defibrillator 40.

### SUMMARY OF THE INVENTION

The present disclosure provides a front-end rescue device and a handheld device whereby a power connection between the devices (e.g., via an electronic cable or direct contact) converts the handheld device into a resuscitation hub for locally or remotely controlling a manual defibrillation, an automatic external defibrillation or a semi-automatic external defibrillation of a heart of a patient based on a rescue protocol and electrocardiogram signals received from the front-end rescue device (e.g., ECG, electrode impedance, etc.) via electrodes attached to the patient.

For purposes of describing and claiming the present disclosure, the term "handheld device" broadly encompasses all compact and portable computing/electronic devices, as known in the art of the present disclosure and hereinafter conceived, that are capable of receiving/transmitting, processing and storing data/information. Non-limiting examples of a handheld device include a mobile/cell phone, a personal data assistant, a tablet and a portable media player.

For purposes of describing and claiming the present disclosure, the term "front-end rescue device" broadly encompasses all battery/power supply-free devices, in accordance with the present disclosure and hereinafter conceived, that are capable of processing electrocardiogram signals representative of cardiac rhythm(s) of a heart of a patient and further capable of delivering a defibrillation shock to the heart of the patient.

The handheld device can further guide a cardiopulmonary resuscitation (CPR) of the patient by a responder based on the rescue protocol and CPR-related signals received from the front-end rescue device (e.g., a compression depth, compression rate, chest release and recoil) via a CPR coaching device being applied by the responder to the patient.

The handheld device can further notify nearby volunteer responder(s) if necessary and contact professional responder(s) via an emergency number (e.g., 911).

The present disclosure can be embodied as (1) a handheld device, (2) a front-end rescue device, (3) a resuscitation system employing the hand-held device and the front-end rescue device, and (4) a resuscitation method executable in combination by the hand-held device and the front-end rescue device.

Various embodiments of a handheld device of the present disclosure are partitioned from a front-end rescue device a conditional delivery of a defibrillation shock to a heart of the patient. The handheld device employs a rescue controller and a battery and/or a power supply.

The rescue controller is configured to (1) receive electrocardiogram signals from an electrocardiogram module of the front-end rescue device when the electrocardiogram module is in power connection with the battery and/or the power supply and is in electrical communication with the heart of the patient, (2) control a charging of a shock module of the front-end rescue device by the battery and/or the power supply when the shock module is in signal communication with the rescue controller and the shock module is in power connection with the battery and/or the power supply, and (3) subsequent to the charging of the shock module and when the shock module is in electrical communication with the heart of the patient, control a conditional discharging of the shock module to the heart of the patient based on the electrocardiogram signals received from the electrocardiogram module.

Various embodiments of a front-end rescue device of the present disclosure are partitioned from a handheld device a conditional delivery of a defibrillation shock to a heart of the patient. The front-end rescue device employs an electrocardiogram module and a shock module.

The electrocardiogram module is configured to communicate electrocardiogram signals to a rescue controller of the handheld device when the electrocardiogram module is power connected to a battery and/or a power supply of the handheld device and in electrical communication with the heart of the patient.

The shock module is configured to (1) be charged by the battery and/or the power supply when the shock module is in signal communication with the rescue controller and is power connected to the battery and/or the power supply, and (2) subsequent to the charging of the shock module and when the shock module is in electrical communication with the heart of the patient, the shock module is further configured to be conditionally discharged to the heart of the patient by the rescue controller based on the electrocardiogram signals received by the rescue controller.

Various embodiments of a resuscitation system of the present disclosure employ a front-end rescue device and a handheld device for a conditional delivery of a defibrillation shock to a heart of the patient.

The front-end rescue device is partitioned from the hand-held device, and includes an electrocardiogram module and a shock module.

The hand-held device includes a rescue controller and a battery and/or a power supply.

The electrocardiogram module is configured to communicate electrocardiogram signals to the rescue controller when the electrocardiogram module is power connected to the battery and/or the power supply and is in electrical communication with the heart of the patient.

The rescue controller is configured to (1) control a charging of the shock module by the battery and/or the power supply when the shock module is in signal communication with the rescue controller and in power connection with the battery and/or the power supply, and (2) subsequent to the charging of the shock module and when the shock module is in electrical communication with the heart of the patient, the rescue controller is further configured to control a conditional discharging of the shock module to the heart of the patient based on the electrocardiogram signals received from the electrocardiogram module.

Various embodiments of a resuscitation method of the present disclosure are executable by a front-end rescue device and a handheld device for a conditional delivery of a defibrillation shock to a heart of the patient.

The front-end rescue device is partitioned from the hand-held device, and includes an electrocardiogram module and a shock module.

The hand-held device includes a rescue controller and a battery and/or a power supply.

The resuscitation method involves (1) the electrocardiogram module communicating electrocardiogram signals to the rescue controller when the electrocardiogram module is power connected to the battery and/or the power supply and is in electrical communication with the heart of the patient, (2) the rescue controller controlling a charging of the shock module by the battery and/or the power supply when the shock module is in signal communication with the rescue controller and in power connection with the battery and/or the power supply, and (3) subsequent to the charging of the shock module and when the shock module is in electrical communication with the heart of the patient, the rescue controller controlling a conditional discharging of the shock module to the heart of the patient based on the electrocardiogram signals received from the electrocardiogram module.

The foregoing exemplary embodiments and other embodiments of the present disclosure as well as various structures and advantages of the present disclosure will become further apparent to those having ordinary skill in the art from the following detailed description of various embodiments of the present disclosure read in conjunction with the accompanying drawings. The detailed description and drawings are merely illustrative of the present disclosure rather than limiting, the scope of the present disclosure being defined by the appended claims and equivalents thereof.

### BRIEF DESCRIPTION OF THE DRAWINGS

The present disclosure will present in detail the following description of exemplary embodiments with reference to the following figures wherein:
FIG. 1 illustrates a cardiopulmonary resuscitation being administered by a responder to a heart of a patient as known in the art of the present disclosure;
FIG. 2 illustrates an exemplary embodiment of a resuscitation system in accordance with the present disclosure;
FIG. 3 illustrates an exemplary embodiment of a rescue module of a handheld device in accordance with the present disclosure;
FIG. 4 illustrates an exemplary embodiment of a rescue protocol in accordance with the present disclosure;
FIG. 5 illustrates an exemplary embodiment of an ECG module of a front-end rescue device in accordance with the present disclosure;
FIG. 6 illustrates an exemplary embodiment of a shock module of a front-end rescue device in accordance with the present disclosure; and
FIG. 7 illustrates flowcharts representative of an exemplary embodiment of a resuscitation method in accordance with the present disclosure.

### DETAILED DESCRIPTION OF THE PREFERRED EMBODIMENTS

The present disclosure provides a front-end rescue device and a handheld device (e.g., a cell phone and a tablet) whereby a power connection between the devices (e.g., via an electronic cable or direct contact) converts the handheld device into a resuscitation hub for locally or remoting controlling a manual defibrillation, an automatic external defibrillation or a semi-automatic external defibrillation of a heart of a patient.

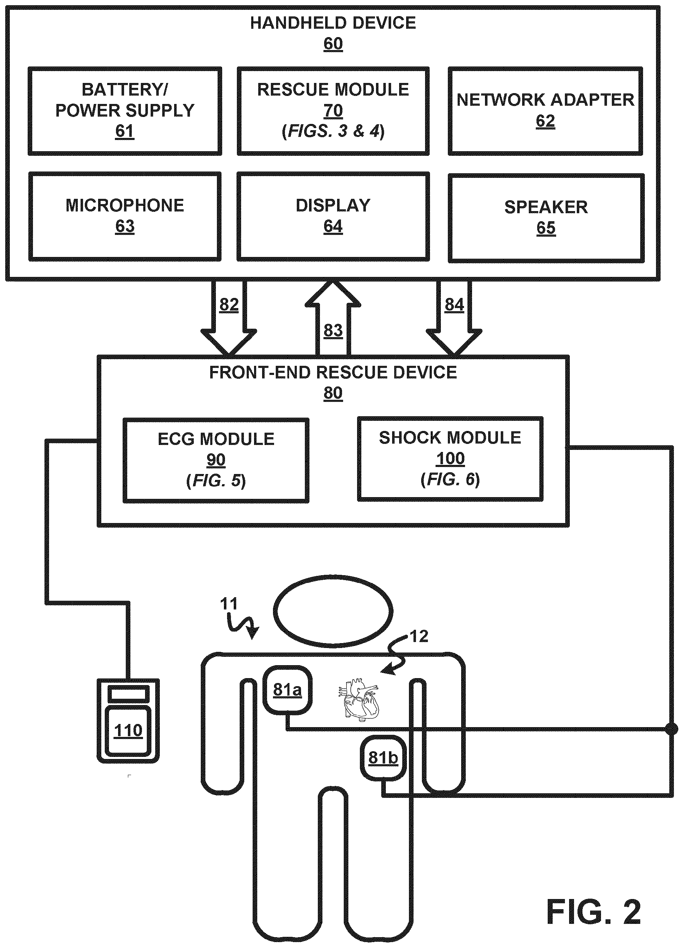

To facilitate an understanding of the present disclosure, the following description of FIGS. 2-7 describes and teaches exemplary embodiments of devices, systems and methods in accordance with the present disclosure. From the description of FIGS. 2-7, those having ordinary skill in the art of the present disclosure will appreciate how to apply the present disclosure to make and use additional embodiments of devices, systems and methods in accordance with the present disclosure.

FIG. 2 illustrates an exemplary resuscitation system of the present disclosure employs a handheld device 60 and a front-end rescue device 80 partitioned from handheld device 80.

Referring to FIG. 2, handheld device 60 is a compact and portable device including a battery/power supply 61, a network adapter 62, a microphone 63, a display 64 and a speaker 65 as known in the art of the present disclosure.

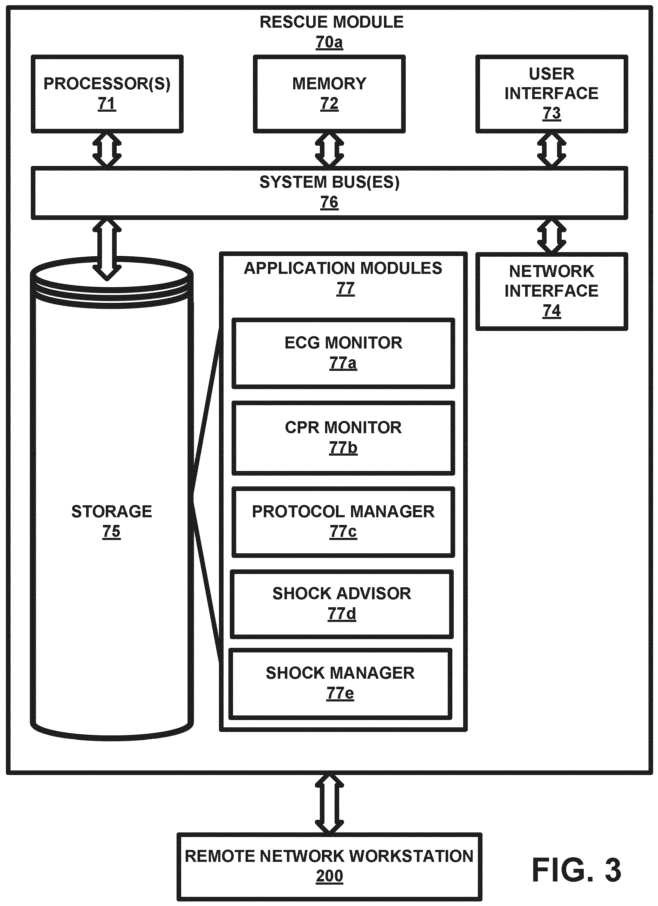

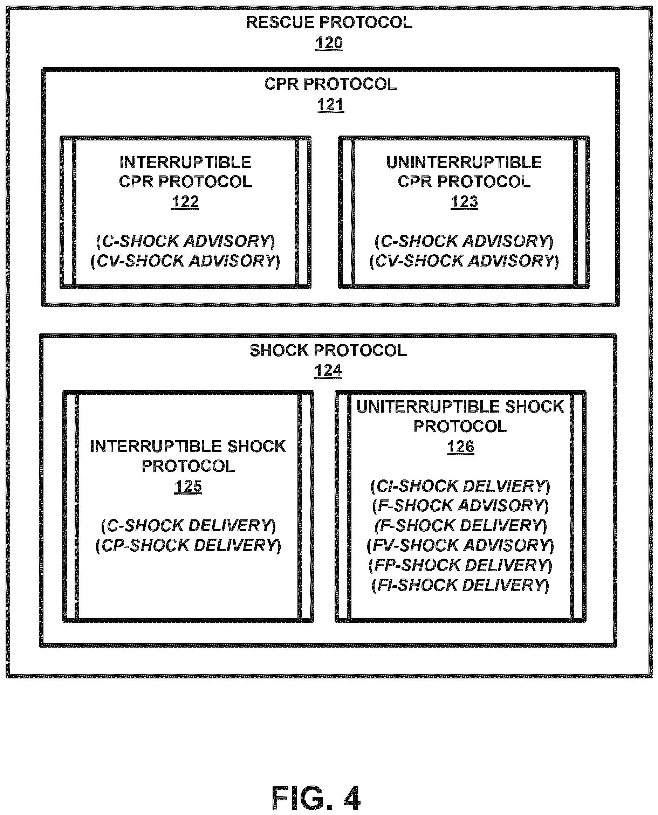

Handheld device 60 further includes a rescue module 70 in accordance with the present disclosure locally or remoting controlling a manual defibrillation, an automatic external defibrillation or a semi-automatic external defibrillation of a heart 12 of a patient 11 as will further described in the description of FIGS. 3 and 4 of the present disclosure.

Still referring to FIG. 2, front-end rescue device is a battery/power supply-free device including an electrocardiogram (ECG) module 90 of the present disclosure and a shock module 100 of the present disclosure.

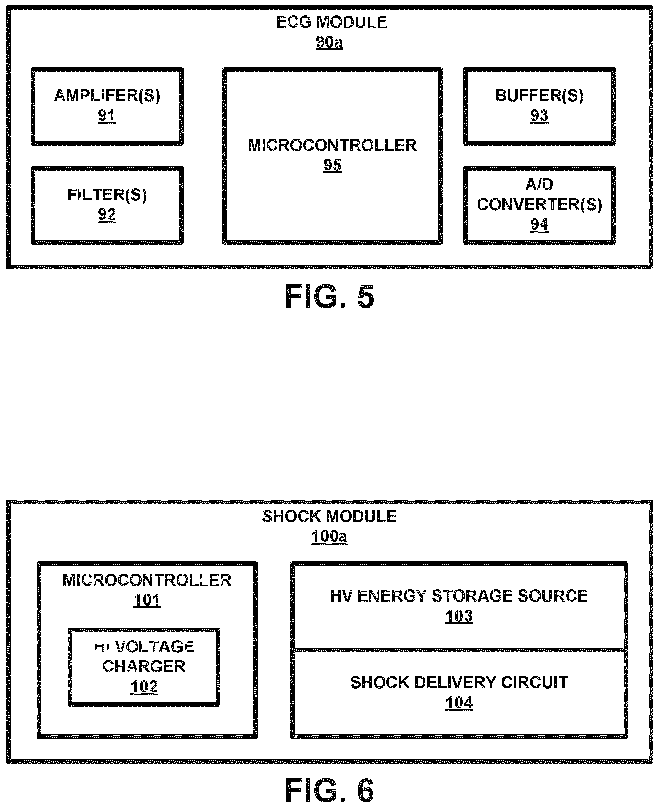

ECG module 90 in accordance with the present disclosure is an electronic circuit (e.g., electronic components and/or hardware) and/or as a controller having an executable program (e.g., executable software stored on non-transitory computer readable medium(s) and/or firmware) for processing electrocardiogram signals representative of cardiac rhythm(s) of heart 12 of patient 10 as will further described in the description of FIG. 5 of the present disclosure.

Shock module 100 in accordance with present disclosure is an electronic circuit (e.g., e.g., electronic components and/or hardware) for delivering a defibrillation shock to the heart 12 of the patient 11 as will further described in the description of FIG. 6 of the present disclosure.

In practice of the present disclosure, a power connection 82 between handheld device 60 and front-end rescue device 80 facilitates a transmission of power from battery/power supply 61 for powering a signal processing by ECG module 90 to receive electrocardiogram signals from electrodes 81a and 81b, and furth for a charging of shock module 100 to a specified level.

A signal connection 83 between handheld device 60 and front-end rescue device 80 facilitates a transmission of the electrocardiogram signals processed by ECG module 90 to rescue module 70.

A signal connection 84 between handheld device 60 and front-end rescue device 80 facilitates a transmission of a shock signal from rescue module 70 to shock module 100 to thereby discharge shocking module for delivery a defibrillation shock to heart 12 of patent 11 via electrodes 81a and 81b.

Further in practice of the present disclosure, power connection 82, signal connection 83 and signal connection 84 can be integrally or individually implemented, as known in the art of the present disclosure, by electronic cable(s), wireless communication device(s) and/or front-end rescue device 80 serving as a docking station for handheld device 60.

Still referring to FIG. 1, ECG module 90 can further receive and process CPR-related signal(s) (e.g., a compression depth, compression rate, chest release and recoil) via a CPR coaching device 110 that is being applied by the responder to patient 11, and transmit the process CPR-related signals to rescue module 70 via signal connection 83 whereby rescue module 70 can utilized display 64 and/or speaker 65 to communicate the CPR-related signal(s) to the responder.

FIG. 3 illustrates an exemplary embodiment of a rescue module 70a including one or more processor(s) 71, memory 72, a user interface 73, a network interface 74, and a storage 75 interconnected via one or more system bus(es) 76.

Referring to FIG. 3, each processor 71 can be any hardware device, as known in the art of the present disclosure or hereinafter conceived, capable of executing instructions stored in memory 72 or storage or otherwise processing data. In a non-limiting example, the processor(s) 71 can include a microprocessor, field programmable gate array (FPGA), application-specific integrated circuit (ASIC), or other similar devices.

The memory 72 can include various memories, as known in the art of the present disclosure or hereinafter conceived, including, but not limited to, L1, L2, or L3 cache or system memory. In a non-limiting example, the memory 72 can include static random access memory (SRAM), dynamic RAM (DRAM), flash memory, read only memory (ROM), or other similar memory devices.

In practice, rescue module 70a also provides control of the user interface (UI) output functions. Specifically, user interface 73 is the primary means for guiding the responder through the protocols of the present disclosure, and so includes at least one of an aural instruction output and a visual display. In particular, user interface 73 may comprise an audio speaker to issue an aural verbal or signal prompt to the responder regarding a state of the rescue, an instruction as to a next step to be taken in the rescue, or regarding instructions responsive to an execution of a particular protocol (e.g., administering CPR and/or delivering a drug). User interface 73 can also convey audible information via a beeper. User interface 73 can also provide visual text or graphical indications on a display. User interface 73 can also convey visual information via a flashing light LED, which may illuminate adjacent graphics or buttons to be pressed. Preferably, rescue module 70a controls the user interface 71 such that each of these cues is provided in a manner that optimizes the desired response of the responder in the execution of protocols of the present disclosure.

Still referring to FIG. 6, network interface 74 can include one or more devices, as known in the art of the present disclosure or hereinafter conceived, for enabling communication with other components of handheld device 60 and with other network devices, as known in the art of the present disclosure or hereinafter conceived.

In a non-limiting example, the network interface 74 can include a network interface card (NIC) configured to communicate according to the Ethernet protocol. Additionally, the network interface 414 may implement a TCP/IP stack for communication according to the TCP/IP protocols. Various alternative or additional hardware or configurations for the network interface 74 will be apparent.

The storage 75 can include one or more machine-readable storage media, as known in the art of the present disclosure or hereinafter conceived, including, but not limited to, read-only memory (ROM), random-access memory (RAM), magnetic disk storage media, optical storage media, flash-memory devices, or similar storage media. In various non-limiting embodiments, the storage 75 can store instructions for execution by the processor(s) 71 or data upon with the processor(s) 71 may operate. For example, the storage 75 may store a base operating system for controlling various basic operations of the hardware.

The storage 75 can also store an application modules 77 in the form of executable software/firmware for implementing the methods of the present disclosure as described in the present disclosure.

In this exemplary embodiment, application modules 77 include a ECG monitor 77a, a CPR monitor 77b, a protocol manager 77c, a shock advisor 77d and a shock manager 77c.

ECG monitor 77a includes instructions for controlling a graphical display of an electrocardiogram derived from ECG signals received from ECG module 90 as known in the art of the present disclosure or hereinafter conceived.

CPR monitor 77b includes instructions for communicating a CPR quality derived from the CPR-related signals received from ECG module 90 as known in the art of the present disclosure or hereinafter conceived.

Protocol manager 77c includes instructions for executing a rescue protocol as known in the art of the present disclosure or hereinafter conceived.

Shock advisor 77d includes instructions for executing a shock advisory based on ECG signals received from ECG module 90 in accordance with the present disclosure.

Shock manager 77e includes instructions for executing a delivery of a defibrillation shock to a heart of patient in accordance with the present disclosure.

Application modules 77 can further include a self-test application to perform periodic self-test to make sure (1) all applications are working as designed, (2) the handheld device is working properly to support all needed functions, and (3) any software updates in the handheld device don't affect the functionalities of rescue module 70. Furthermore, the self-test application can have weekly or monthly reminders to let the user connect the front-end rescue device to perform test.

Application modules 77 can further include a battery level application to ensure a battery of the handheld device is sufficiently charged for a rescue. In practice, a10% battery level of the handheld device should be enough for several shocks, and when the battery level is lower than a certain threshold, the battery level application issues an alarm to let the user to charge the handheld device.

In an alternative embodiment, application modules 77 can be alternatively or concurrently programed in a remote network workstation 200 whereby the processing of the ECG signals and the generation of the shock signal can alternatively be performed by the remote network workstation 200.

Referring to FIG. 3, in practice of the present disclosure, rescue module 70 can be a stand-alone module segregated from other components of the handheld device, or can be integrated with other processing component(s) of the handheld device.

FIG. 4 illustrates an exemplary embodiment of a rescue protocol 120 including a CPR protocol 121 and a shock protocol 124.

Referring to FIGS. 3 and 4, protocol manager 77c is programmed to execute rescue protocol 120 accordance with rule(s)/guideline(s) (e.g., rule(s)/guideline(s) established by the American Heart Association). In a non-liming example, rescue protocol 120 implements a custom operation mode, a schedule operation mode or a combination thereof.

Shock advisor 77d is programmed to execute one or more shock advisories of CPR protocol 121 and shock protocol 124 dependent upon the rule(s)/guidelines of rescue protocol 120, and shock manager 77e is programmed to execute one or more shock delivers a shock protocol 124 dependent upon the rule(s)/guidelines of rescue protocol 120.

For purposes of describing and claiming the present disclosure,
(1) terms of the art of the present disclosure, but not limited to, "defibrillating shock", "electrocardiogram (ECG)", "cardiac rhythm", "cardiopulmonary resuscitation (CPR)", "rescue protocol", "CPR protocol" and "shock protocol", are to be interpreted as known in the art of the present disclosure and as exemplary described in the present disclosure;
(2) the term "C-Shock Advisory" broadly encompasses all methods, as known in the art of the present disclosure or hereinafter conceived, for analyzing and classifying a cardiac rhythm of an ECG of a heart of a patient including artifacts as known in the art of the present disclosure resulting from an administration of chest compressions to the heart of the patient (i.e., a corrupt ECG) to thereby derive
   (a) a shock decision based upon a determinate classification of the corrupt ECG as having a shockable cardiac rhythm (e.g., a ventricular fibrillation (VF) rhythm or a ventricular tachycardia (VT) rhythm),
   (b) a non-shock decision based upon a determinate classification of the corrupt ECG as having a non-shockable cardiac rhythm (e.g., a pulseless electrical activity rhythm or an asystole rhythm), or
   (c) an undecided shock decision based upon an indeterminate classification of the corrupt ECG as having a shockable cardiac rhythm (e.g., a ventricular fibrillation (VF) rhythm or a ventricular tachycardia (VT) rhythm) or a non-shockable cardiac rhythm (e.g., a pulseless electrical activity rhythm or an asystole rhythm);
(3) the term "C-Shock Delivery" broadly encompasses all methods, as known in the art of the present disclosure or hereinafter conceived, for delivering a defibrillating shock to a heart of a patient corresponding to a shock decision derived by the C-Shock Advisory in accordance with rule(s)/guideline(s) associated with a rescue protocol (e.g., rule(s)/guideline(s) established by the American Heart Association);
(4) the term "F-Shock Advisory" broadly encompasses all methods, as known in the art of the present disclosure or hereinafter conceived, for analyzing and classifying a cardiac rhythm of an ECG of a heart of a patient excluding artifacts as known in the art of the present disclosure resulting from a termination/suspension of an administration of chest compressions to the heart of the patient (i.e., a clean ECG) to thereby derive
   (a) a shock decision based upon a determinate classification of the clean ECG as having a shockable cardiac rhythm (e.g., a ventricular fibrillation (VF) rhythm or a ventricular tachycardia (VT) rhythm),
   (b) a non-shock decision based upon a determinate classification of the clean ECG as having a non-shockable cardiac rhythm (e.g., a pulseless electrical activity rhythm or an asystole rhythm, or
   (c) an undecided shock decision based upon an indeterminate classification of the clean ECG as having a shockable cardiac rhythm (e.g., a ventricular fibrillation (VF) rhythm or a ventricular tachycardia (VT) rhythm) or a non-shockable cardiac rhythm (e.g., a pulseless electrical activity rhythm or an asystole rhythm);
(5) the term "F-Shock Delivery" broadly encompasses all methods, as known in the art of the present disclosure or hereinafter conceived, for delivering a defibrillating shock to a heart of a patient corresponding to a shock decision derived by the F-Shock Advisory in accordance with rule(s)/guideline(s) associated with a rescue protocol (e.g., rule(s)/guideline(s) established by the American Heart Association); and
(6) the term "CPR Quality Analysis" broadly encompasses all methods, as known in the art of the present disclosure or hereinafter conceived, for monitoring a quality of CPR being administered to a heart of a patient to derive an assessment of a high quality/compliant CPR or a low quality/non-compliant CPR.

A non-limiting example of a C-Shock Advisory is an Arrythmia Recognition Technology (ART) as known in the art of the present disclosure.

A non-limiting example a F-Shock Advisory is a Patient Analysis System (PAS) as known in the art of the present disclosure.

In practice, for the C-Shock Advisory and the F-Shock Advisory, one or more of the described shock decisions can be omitted and/or additional shock decision(s) can be derived from an analysis and rhythm classification of ECG.

A non-limiting example of CPR Quality Analysis is a monitoring of a depth of compression, a rate of compression and chest release and recoil effective relative to rule(s)/guideline(s) of a rescue protocol (e.g., rule(s)/guideline(s) established by the American Heart Association) to derive an assessment of a high quality/compliant CPR being administered to the heart of the patient or a low quality/non-compliant CPR being administered to the heart of the patient.

An additional non-limiting example of CPR Quality Analysis is an implementation of a physiological sensor/monitor for measuring blood flow, cerebral perfusion and/or myocardial perfusion.

Also for purposes of describing and claiming the present disclosure,
(1) the term "rescue outcome" broadly encompasses a defibrillation of a heart of a patient with or without resuscitation of the patient (e.g., a return of an organized cardiac rhythm with or without a sustained return of spontaneous circulation) and further broadly encompasses a resuscitation of the patient (e.g., a defibrillation of the heart with a sustained return of spontaneous circulation or a survival of the patient with a cerebral performance category 1 or a cerebral performance category 2);
(2) the term "probable rescue outcome" broadly encompasses a probability of a defibrillation of a heart derived, directly or indirectly, from a successful restoration of an organized rhythm and contractile function of a heart from a delivery of a defibrillating shock to the heart of the patient;
(3) the term "improbable rescue outcome" broadly encompasses an improbability of a defibrillation of the heart derived, directly or indirectly, from an unsuccessful restoration of an organized rhythm and contractile function of the heart from a delivery of a defibrillation shock to the heart of the patient;
(4) the term "probable defibrillating shock" broadly encompasses a defibrillating shock delineated as having a probable rescue outcome;
(5) the term "improbable defibrillating shock" broadly encompasses a defibrillating shock delineated as having an improbable rescue outcome;
(6) the term "CV-Shock Advisory" broadly encompasses all methods, as known in the art of the present disclosure or hereinafter conceived, for analyzing and classifying a cardiac rhythm of an ECG of a heart of a patient including artifacts as known in the art of the present resulting from an administration of chest compressions to the heart of the patient (i.e., a corrupt ECG) and for predicting a probable rescue outcome or an improbable rescue outcome to thereby derive
   (a) a CP-shock decision based upon a determinate classification of the corrupt ECG as having a shockable cardiac rhythm (e.g., a ventricular fibrillation (VF) rhythm or a ventricular tachycardia (VT) rhythm) and based further upon a prediction of a probable rescue outcome,
   (b) a CI-shock decision based upon a determinate classification of the corrupt ECG as having a shockable cardiac rhythm (e.g., a ventricular fibrillation (VF) rhythm or a ventricular tachycardia (VT) rhythm) and based further upon a prediction of an improbable rescue outcome,
   (c) a non-shock decision based upon a determinate classification of the corrupt ECG as having a non-shockable cardiac rhythm (e.g., a pulseless electrical activity rhythm or an asystole rhythm, or
   (d) an undecided shock decision based upon an indeterminate classification of the corrupt ECG as having a shockable cardiac rhythm (e.g., a ventricular fibrillation (VF) rhythm or a ventricular tachycardia (VT) rhythm) or a non-shockable cardiac rhythm (e.g., a pulseless electrical activity rhythm or an asystole rhythm);
(7) the term "CP-Shock Delivery" broadly encompasses all methods, as known in the art of the present disclosure or hereinafter conceived, for delivering a defibrillating shock to a heart of a patient corresponding to a CP-shock decision derived by the CV-Shock Advisory in accordance with rule(s)/guideline(s) associated with a rescue protocol (e.g., rule(s)/guideline(s) established by the American Heart Association);
(8) the term "CI-Shock Delivery" broadly encompasses all methods, as known in the art of the present disclosure or hereinafter conceived, for delivering a defibrillating shock to a heart of a patient corresponding to a CI-shock decision derived by the CV-Shock Advisory in accordance with rule(s)/guideline(s) associated with a rescue (e.g., rule(s)/guideline(s) established by the American Heart Association);
(9) the term "FV-Shock Advisory" broadly encompasses all methods, as known in the art of the present disclosure or hereinafter conceived, for analyzing and classifying a cardiac rhythm of an ECG of a heart of a patient excluding artifacts as known in the art of the present disclosure resulting from an administration of chest compressions to the heart of the patient (i.e., a clean ECG) and for predicting a probable rescue outcome or an improbable rescue outcome to thereby derive
   (a) a FP-shock decision based upon a determinate classification of the clean ECG as having a shockable cardiac rhythm (e.g., a ventricular fibrillation (VF) rhythm or a ventricular tachycardia (VT) rhythm) and upon a prediction of a probable rescue outcome,
   (b) a FI-shock decision based upon a determinate classification of the clean ECG as having a shockable cardiac rhythm (e.g., a ventricular fibrillation (VF) rhythm or a ventricular tachycardia (VT) rhythm) and upon a prediction of an improbable rescue outcome,
   (c) a non-shock decision based upon a determinate classification of the clean ECG as having a non-shockable cardiac rhythm (e.g., a pulseless electrical activity rhythm or an asystole rhythm, or
   (d) an undecided shock decision based upon an indeterminate classification of the clean ECG as having a shockable cardiac rhythm (e.g., a ventricular fibrillation (VF) rhythm or a ventricular tachycardia (VT) rhythm) or a non-shockable cardiac rhythm (e.g., a pulseless electrical activity rhythm or an asystole rhythm);
(10) the term "FP-Shock Delivery" broadly encompasses all methods, as known in the art of the present disclosure or hereinafter conceived, for delivering a defibrillating shock to a heart of a patient corresponding to a FP-shock decision derived by the FV-Shock Advisory in accordance with rule(s)/guideline(s) associated with a rescue protocol (e.g., rule(s)/guideline(s) established by the American Heart Association); and
(11) the term "FI-Shock Delivery" broadly encompasses all methods, as known in the art of the present disclosure or hereinafter conceived, for delivering a defibrillating shock to a heart of a patient corresponding to a FI-shock decision derived by the FV-Shock Advisory in accordance with rule(s)/guideline(s) associated with a rescue protocol (e.g., rule(s)/guideline(s) established by the American Heart Association).

A non-limiting example of a FV-Shock Advisory is a Patient Analysis System (PAS) as known in the art of the present disclosure incorporating a measurement of a vitality of a shockable cardiac rhythm as known in the art of the present disclosure (e.g., a measurement of an amplitude/frequency of the shockable cardiac rhythm relative to a sensitivity/specificity based threshold delineating between a probable rescue outcome or an improbable rescue outcome).

A non-limiting example of a CV-Shock Advisory is an Arrythmia Recognition Technology (ART) as known in the art of the present disclosure incorporating a measurement of a vitality of a shockable cardiac rhythm as known in the art of the present disclosure (e.g., a measurement of an amplitude/frequency of the shockable cardiac rhythm relative to a sensitivity/specificity based threshold delineating between a probable rescue outcome or an improbable rescue outcome).

In practice, for the CV-Shock Advisory and the FV-Shock Advisory, one or more of the described shock decisions can be omitted and/or additional shock decision(s) can be derived from an analysis and classification of a cardiac rhythm of an ECG.

FIG. 5 illustrates an exemplary embodiment 90a of ECG module 90 (FIG. 2).

Referring to FIG. 2, ECG module 90a includes amplifier(s) 91, filter(s) 92, buffer(s) 93 and analog-to-digital converter(2) 94 as known in the art of the present disclosure for amplifying, filtering, buffering and digitizing ECG signals received from electrodes.

ECG module 90 can further includes a microcontroller 95 for generating ECG-related signals as known in the art of the present disclosure, such as, for example, an impedance between the electrodes and for controlling the communications of signals from the electrodes and to rescue module 70 as known in the art of the present disclosure.

FIG. 6 illustrates an exemplary embodiment 100a of shock module 100 (FIG. 2).

Referring to FIG. 6, shock module 100a includes a microcontroller 101, High Voltage (HV) energy storage source 103 and a shock delivery circuit 104. In operation, microcontroller 101 receives a charge signal from rescue module 70 (FIG. 2) to thereby charge HV energy storage source 102 in preparation for delivering a shock. When the HV energy storage source 102 is fully charged and upon a shock signal from rescue module 70, microcontroller 101 activates a shock delivery circuit to discharge HV energy storage source 104.

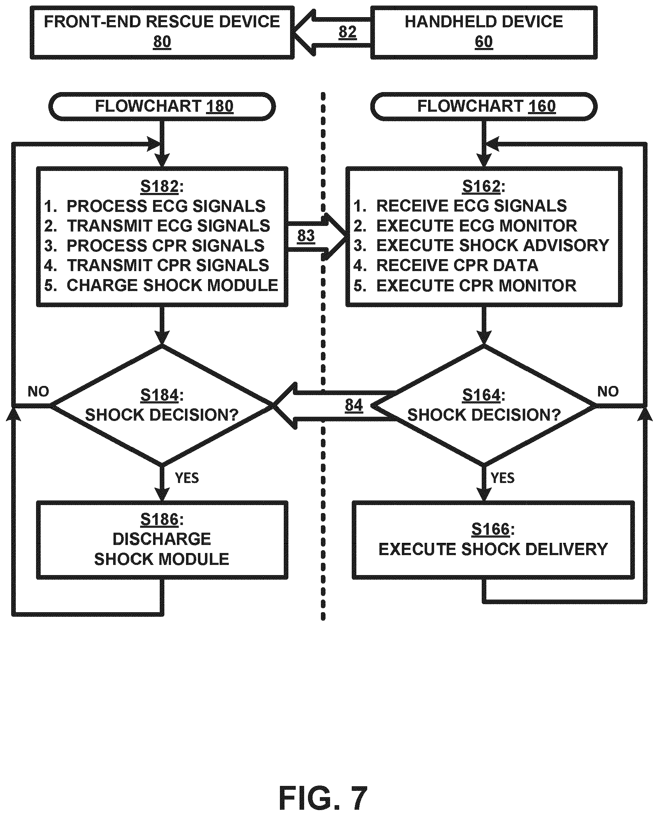

FIG. 7 illustrates a flowchart 160 and a flowchart 180 representative of a resuscitation method of the present disclosure. In practice, flowchart 160 is executed by handheld device 60 and flowchart 180 is executed by front-end rescue device 80 upon a power connection 82 between the devices.

Referring to FIG. 7, a stage S 182 of flowchart 180 encompasses ECG module 90 of front-end rescue device 80 (1) processing ECG signals from the electrodes, (2) transmitting the ECG signals to rescue module 70 of handheld device 60, (3) processing CPR signals from CPR coaching device, and (4) transmitting the CPR signals to rescue module 70.

Stage S 182 of flowchart 180 further encompasses a charging of shock module 100.

A stage S162 of flowchart 160 encompasses rescue module 70 (1) receiving ECG signals from ECG module 90, (2) if applicable, an execution of a shock advisory of the ECG signals in dependence of the rescue protocol and the type of defibrillation (e.g., manual, automatic or semi-automatic), (3) if applicable, an execution of a ECG monitor of the ECG signals in dependence of the rescue protocol and the type of defibrillation (e.g., manual, automatic or semi-automatic), (receiving CPR signals from ECG module 90, (4) receiving CPR signals from ECG module 90 and (5) execute the CPR monitor.

If the execution of the shock advisory during stage S162 results in a shock decision, then a stage S 164 of flowchart 160 will communicate a shock signal to shock module 100 whereby shock module will proceed from a stage S 184 of flowchart 180 to a stage S 186 to discharge the shock module.

Upon a discharging of the shock module, stages 162 and 182 are repeated.

From the description of FIGS. 2-7 herein, those having ordinary skill in the art will appreciate the numerous benefits of the present disclosure including, but not limited to, a conversion of the handheld device into a resuscitation hub for locally or remotely controlling a manual defibrillation, an automatic external defibrillation or a semi-automatic external defibrillation of a heart of a patient based on a rescue protocol and electrocardiogram signals received from the front-end rescue device (e.g., ECG, electrode impedance, etc.) via electrodes attached to the patient.

The present disclosure has been described with reference to the preferred embodiments. Modifications and alterations may occur to others upon reading and understanding the preceding detailed description. It is intended that the invention be construed as including all such modifications and alterations insofar as they come within the scope of the appended claims or the equivalents thereof.

Further, as one having ordinary skill in the art shall appreciate in view of the teachings provided herein, features, elements, components, etc. disclosed and described in the present disclosure/specification and/or depicted in the appended Figures and/or recited in the Claims can be implemented in various combinations of hardware and software, and provide functions which can be combined in a single element or multiple elements. For example, the functions of the various features, elements, components, etc. shown/illustrated/depicted in the Figures and/or recited in the Claims can be provided through the use of dedicated hardware as well as hardware capable of executing software in association with appropriate software. When provided by a processor, the functions can be provided by a single dedicated processor, by a single shared processor, or by a plurality of individual processors, some of which can be shared and/or multiplexed. Moreover, explicit use of the term "processor" or "controller" should not be construed to refer exclusively to hardware capable of executing software, and can implicitly include, without limitation, digital signal processor ("DSP") hardware, memory (e.g., read only memory ("ROM") for storing software, random access memory ("RAM"), non-volatile storage, etc.) and virtually any means and/or machine (including hardware, software, firmware, combinations thereof, etc.) which is capable of (and/or configurable) to perform and/or control a process.

Moreover, all statements herein reciting principles, aspects, and exemplary embodiments of the present disclosure, as well as specific examples thereof, are intended to encompass both structural and functional equivalents thereof. Additionally, it is intended that such equivalents include both currently known equivalents as well as equivalents developed in the future (e.g., any elements developed that can perform the same or substantially similar functionality, regardless of structure). Thus, for example, it will be appreciated by one having ordinary skill in the art in view of the teachings provided herein that any block diagrams presented herein can represent conceptual views of illustrative system components and/or circuitry embodying the principles of the invention. Similarly, one having ordinary skill in the art should appreciate in view of the teachings provided herein that any flow charts, flow diagrams and the like can represent various processes which can be substantially represented in computer readable storage media and so executed by a computer, processor or other device with processing capabilities, whether or not such computer or processor is explicitly shown.

Having described preferred and exemplary embodiments of the present disclosure, which embodiments are intended to be illustrative and not limiting, it is noted that modifications and variations can be made by persons having ordinary skill in the art in view of the teachings provided herein, including the appended Figures and claims. It is therefore to be understood that changes can be made in/to the preferred and exemplary embodiments of the present disclosure which are within the scope of the present disclosure and exemplary embodiments disclosed, described and taught herein.

Moreover, it is contemplated that corresponding and/or related systems incorporating and/or implementing the device or such as can be used/implemented in a device in accordance with the present disclosure are also contemplated and considered to be within the scope of the present disclosure. Further, corresponding and/or related method for manufacturing and/or using a device and/or system in accordance with the present disclosure are also contemplated and considered to be within the scope of the present disclosure.

## Claims

1. A defibrillation controller (204) for controlling an administration of a cardiopulmonary resuscitation procedure on a heart of a patient by a defibrillator (200) in accordance with a rescue protocol including an interruptible cardiopulmonary resuscitation protocol, an uninterruptible cardiopulmonary resuscitation protocol and a shock protocol,
the interruptible cardiopulmonary resuscitation protocol specifying at least one rule/guideline for a guidance by the defibrillator (200) of an administration of a cardiopulmonary resuscitation to the heart of the patient over a terminable CPR time period,
the interruptible cardiopulmonary resuscitation protocol specifying at least one rule/guideline for a guidance by the defibrillator (200) of an administration of a cardiopulmonary resuscitation to the heart of the patient over a terminable CPR time period,
the shock protocol specifying at least one rule/guideline for a conditional delivery of an interrupted defibrillating shock and for a conditional delivery of an uninterrupted defibrillating shock to the heart of the patient by the defibrillator (200),
the defibrillation controller (204) comprising a non-transitory machine-readable storage medium encoded with instructions for execution by at least one processor, the non-transitory machine-readable storage medium including the instructions to:
control a series of sequential executions of the interruptible cardiopulmonary resuscitation protocol and the shock protocol in response to a preceding uninterrupted defibrillating shock delivery to the heart of the patient; and
control a sequential execution of the uninterruptible cardiopulmonary resuscitation protocol and the shock protocol when at least two sequential executions of the interruptible cardiopulmonary resuscitation protocol and the shock protocol results in a maximum number of consecutive interrupted defibrillating shock deliveries to the heart of the patient.

2. The defibrillation controller (204) of claim 1,
wherein the interruptible cardiopulmonary resuscitation protocol includes a minimum non-shock interval of the terminal CPR time period; and
wherein the non-transitory machine-readable storage medium further includes instructions to:
control an initiation of an execution of the shock protocol of each sequential execution of the interruptible cardiopulmonary resuscitation protocol and the shock protocol based on the minimum non-shock interval of the interruptible cardiopulmonary resuscitation protocol.

3. The defibrillation controller (204) of claim 1, wherein the non-transitory machine-readable storage medium further includes instructions to:
control an additional series of sequential executions of the interruptible cardiopulmonary resuscitation protocol and the shock protocol when the sequential execution of the uninterruptible cardiopulmonary resuscitation protocol and the shock protocol results in an additional uninterrupted defibrillating shock delivery to the heart of the patient.

4. The defibrillation controller (204) of claim 1, wherein the non-transitory machine-readable storage medium further includes instructions to:
control at least one additional sequential execution of the uninterruptible cardiopulmonary resuscitation protocol and the shock protocol when the sequential execution of the uninterruptible cardiopulmonary resuscitation protocol and the shock protocol does not result in an additional uninterrupted defibrillating shock delivery to the heart of the patient.

5. The defibrillation controller (204) of claim 3, wherein the non-transitory machine-readable storage medium further includes instructions to:
control an additional series of sequential executions of the interruptible cardiopulmonary resuscitation protocol and the shock protocol when the at least one additional sequential execution of the uninterruptible cardiopulmonary resuscitation protocol and the shock protocol results in an additional uninterrupted defibrillating shock delivery to the heart of the patient.

6. A defibrillator (200), comprising:
a shock delivery circuit (207) operable to deliver at least one defibrillating shock to a heart of a patient; and
a defibrillation controller (204) for controlling an administration of a cardiopulmonary resuscitation procedure on a heart of a patient by the defibrillator (200) in accordance with a rescue protocol including an interruptible cardiopulmonary resuscitation protocol, an uninterruptible cardiopulmonary resuscitation protocol and a shock protocol,
wherein the interruptible cardiopulmonary resuscitation protocol specifies at least one rule/guideline for a guidance by the defibrillator (200) of an administration of a cardiopulmonary resuscitation to the heart of the patient over a terminable CPR time period,
wherein the interruptible cardiopulmonary resuscitation protocol specifies at least one rule/guideline for a guidance by the defibrillator (200) of an administration of a cardiopulmonary resuscitation to the heart of the patient over a terminable CPR time period,
wherein the shock protocol specifies at least one rule/guideline for a conditional delivery of an interrupted defibrillating shock and for a conditional delivery of a uninterrupted defibrillating shock to the heart of the patient by the shock delivery circuit (207), and
wherein the defibrillation controller (204) is configured to:
control a series of sequential executions of the interruptible cardiopulmonary resuscitation protocol and the shock protocol by the defibrillator (200) in response to a preceding uninterrupted defibrillating shock delivery to the heart of the patient by the shock delivery circuit (207); and
control a sequential execution of the uninterruptible cardiopulmonary resuscitation protocol and the shock protocol by the defibrillator (200) when at least two sequential executions of the interruptible cardiopulmonary resuscitation protocol and the shock protocol by the defibrillator (200) results in a maximum number of consecutive interrupted defibrillating shock deliveries to the heart of the patient by the shock delivery circuit (207).

7. The defibrillator (200) of claim 6,
wherein the interruptible cardiopulmonary resuscitation protocol includes a minimum non-shock interval of the terminal CPR time period; and
wherein the defibrillation controller (204) is further configured to:
control an initiation of an execution of the shock protocol of each sequential execution of the interruptible cardiopulmonary resuscitation protocol and the shock protocol based on the minimum non-shock interval of the interruptible cardiopulmonary resuscitation protocol.

8. The defibrillator (200) of claim 6, wherein the defibrillation controller (204) is further configured to:
control an additional series of sequential executions of the interruptible cardiopulmonary resuscitation protocol and the shock protocol when the sequential execution of the uninterruptible cardiopulmonary resuscitation protocol and the shock protocol results in an additional uninterrupted defibrillating shock delivery to the heart of the patient by the shock delivery circuit (207).

9. The defibrillator (200) of claim 6, wherein the defibrillation controller (204) is further configured to:
control at least one additional sequential execution of the uninterruptible cardiopulmonary resuscitation protocol and the shock protocol when the sequential execution of the uninterruptible cardiopulmonary resuscitation protocol and the shock protocol does not result in an additional uninterrupted defibrillating shock delivery to the heart of the patient by the shock delivery circuit (207).

10. The defibrillator (200) of claim 9, wherein the defibrillation controller (204) is further configured to:
control an additional series of sequential executions of the interruptible cardiopulmonary resuscitation protocol and the shock protocol when the at least one additional sequential execution of the uninterruptible cardiopulmonary resuscitation protocol and the shock protocol results in an additional uninterrupted defibrillating shock delivery to the heart of the patient by the shock delivery circuit (207).

11. A defibrillation method executable by a defibrillation controller (204) for controlling an administration of a cardiopulmonary resuscitation procedure on a heart of a patient by a defibrillator (200) in accordance with a rescue protocol including an interruptible cardiopulmonary resuscitation protocol, an uninterruptible cardiopulmonary resuscitation protocol and a shock protocol,
the interruptible cardiopulmonary resuscitation protocol specifying at least one rule/guideline for a guidance by the defibrillator (200) of an administration of a cardiopulmonary resuscitation to the heart of the patient over a terminable CPR time period,
the interruptible cardiopulmonary resuscitation protocol specifying at least one rule/guideline for a guidance by the defibrillator (200) of an administration of a cardiopulmonary resuscitation to the heart of the patient over a terminable CPR time period,
the shock protocol specifying at least one rule/guideline for a conditional delivery of an interrupted defibrillating shock and for a conditional delivery of a uninterrupted defibrillating shock to the heart of the patient by the defibrillator (200),
the defibrillation method comprising:
controlling, by the defibrillation controller (204), a series of sequential executions of the interruptible cardiopulmonary resuscitation protocol and the shock protocol by the defibrillator (200) in response to a preceding uninterrupted defibrillating shock delivery to the heart of the patient; and
controlling, by the defibrillation controller (204), a sequential execution of the uninterruptible cardiopulmonary resuscitation protocol and the shock protocol by the defibrillator (200) when at least two sequential executions of the interruptible cardiopulmonary resuscitation protocol and the shock protocol by the defibrillator (200) results in a maximum number of consecutive interrupted defibrillating shock deliveries to the heart of the patient.

12. The defibrillator method of claim 11,
wherein the interruptible cardiopulmonary resuscitation protocol includes a minimum non-shock interval of the terminal CPR time period; and
further comprising:
controlling, by the defibrillation controller (204), an initiation of an execution of the shock protocol of each sequential execution of the interruptible cardiopulmonary resuscitation protocol and the shock protocol based on the minimum non-shock interval of the interruptible cardiopulmonary resuscitation protocol.

13. The defibrillation method of claim 11, further comprising:
controlling, by the defibrillation controller (204), an additional series of sequential executions of the interruptible cardiopulmonary resuscitation protocol and the shock protocol when the sequential execution of the uninterruptible cardiopulmonary resuscitation protocol and the shock protocol results in an additional uninterrupted defibrillating shock delivery to the heart of the patient

14. The defibrillation method of claim 11,
controlling, by the defibrillation controller (204), at least one additional sequential execution of the uninterruptible cardiopulmonary resuscitation protocol and the shock protocol when the sequential execution of the uninterruptible cardiopulmonary resuscitation protocol and the shock protocol does not result in an additional uninterrupted defibrillating shock delivery to the heart of the patient.

15. The defibrillation method of claim 14,
controlling, by the defibrillation controller (204), an additional series of sequential executions of the interruptible cardiopulmonary resuscitation protocol and the shock protocol when the at least one additional sequential execution of the uninterruptible cardiopulmonary resuscitation protocol and the shock protocol results in an additional uninterrupted defibrillating shock delivery to the heart of the patient.
